# EUROPEAN PATENT APPLICATION

(11) **EP 4 443 571 A1**
(43) Date of publication of application: **09.10.2024**
(21) Application number: 22911124.0
(22) Date of filing: 16.12.2022
(51) Int. Cl.: H01M 8/103, H01B 1/06, H01M 4/86, H01M 4/90, H01M 4/92, H01M 8/10, H01M 8/1018, H01M 12/08

(54) **ELECTROLYTE**

(30) Priority: 22.12.2021 JP 2021208273
(71) Applicant: National Institute of Advanced Industrial Science and Technology, Chiyoda-ku Tokyo 100-8921 (JP)
(72) Inventor: YAMAZAKI, Shin-ichi, Ikeda-shi, Osaka 563-8577 (JP); ASAHI, Masafumi, Ikeda-shi, Osaka 563-8577 (JP); IOROI, Tsutomu, Ikeda-shi, Osaka 563-8577 (JP)
(74) Representative: Jones, Nicholas Andrew
(86) International application number: PCT/JP2022/046410
(87) International publication number: WO 2023/120421

(57) **Abstract**

This electrolyte, which contains a melamine compound and/or a polymer in which said melamine compound is used as a monomer, can effectively suppress the deterioration of a platinum catalyst in an air electrode.

## Description

### TECHNICAL FIELD

The present invention relates to an electrolyte.

### BACKGROUND ART

Polymer electrolyte fuel cells (PEFCs) are compact and efficient and are expected to become widespread from the viewpoint of global environmental problems.

In general, since a polymer electrolyte used in the PEFC is a strongly acidic cation exchange film, it is necessary for an electrode catalyst to stably function under strongly acidic conditions. For this reason, the electrode catalyst that can withstand practical use is only platinum or an alloy thereof at the present point in time. However, since the oxygen reduction reaction at the air electrode is slow, overvoltage is large; thus, a large amount of platinum is used in order to obtain a sufficient reaction speed. It is, however, considered to be an issue that durability of the platinum catalyst of the air electrode is poor.

As a method of reducing such large overvoltage, a method of alloying platinum, a method of using core-shell nanoparticles including platinum, or the like are also known (for example, see

Patent Document 1). There is also known a method of reducing overvoltage by modifying a platinum surface with a tetraazaporphyrin compound (for example, see Patent Document 2).

However, even though durability at room temperature is exhibited in conventional methods as disclosed in Patent Documents 1 and 2, durability at 70 to 85°C, which is a practical temperature condition, cannot be said to be sufficient. The durability at 70 to 85°C, which is a practical temperature condition, has room for improvement and the platinum catalyst deteriorates due to long-term use in PEFC and load fluctuation.

In order to improve the durability of PEFCs, the particle diameter of platinum was increased to about 5 nm, or the upper limit potential of load variation was lowered to about 0.9 V in prior art. However, in the former case, an amount of platinum used increases, and in the latter case a complicated control system is required. Both the above-mentioned countermeasures incur higher costs and there is a need for a countermeasure capable of more efficiently preventing the platinum catalyst from deteriorating.

Incidentally, metal-air batteries are batteries in which a metal such as zinc, iron, aluminum, or the like is used as a negative electrode and an air electrode is used as a positive electrode. These batteries can use oxygen in the air as a positive electrode active material and since the electric capacity is determined only by the negative electrode capacity, a high energy density can be realized. Also in the metal-air batteries, it is known that the reaction on the air electrode (cathode electrode) side is an oxygen reduction reaction during discharge and an oxygen generation reaction during charge. Therefore, since an air electrode using oxygen as an active material is used in the metal-air batteries as in the case of PEFCs or the like, it is required to efficiently suppress deterioration of the platinum catalyst.

### Citation List

### Patent Document

Patent Document 1: Japanese Unexamined Patent Application, Publication No. 2011-092940
Patent Document 2: Japanese Unexamined Patent Application, Publication No. 2018-086640

### DISCLOSURE OF THE INVENTION

### Problems to be Solved by the Invention

It is an object of the present invention to efficiently suppress deterioration of a platinum catalyst in an air electrode.

### Means for Solving the Problems

In view of the above issues, the present inventors have conducted extensive research. As a result, the present inventors have found the following: without adding any modification to the catalyst of the air electrode, by blending a melamine compound or a polymer thereof with the electrolyte, the electrolyte of the present invention does not function as a protective film of a platinum catalyst but suppresses oxidation of the platinum catalyst and can function as an oxidation inhibitor for an electrode electrochemical oxygen reduction catalyst, which increases the particle diameter of the platinum catalyst; as a result, it is possible to suppress a particle diameter of the platinum catalyst from increasing during charge and discharge, resulting in suppression of a surface area from decreasing during charge and discharge, thereby improving durability of a fuel cell or a metal-air battery. Based on such findings, the inventors have continued studies and have arrived at the completion of the present invention. That is, the present invention includes the following configurations.

Item 1. An electrolyte including a melamine compound and/or a polymer including the melamine compound as a monomer.

Item 2. The melamine compound according to item 1, in which the melamine compound is represented by a general formula (1) : 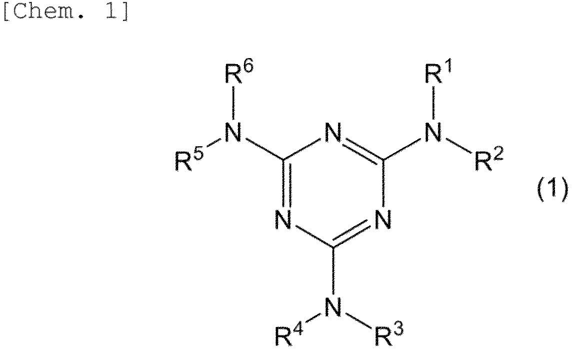 [in the formula, R¹, R², R³, R⁴, R⁵, and R⁶ are the same or different and each represents a hydrogen atom, a hydroxy group, a halogen atom, a substituted or unsubstituted alkyl group, or a substituted or unsubstituted alkenyl group].

Item 3. The electrolyte according to item 1 or 2, in which the electrolyte is an electrolytic solution.

Item 4. The electrolyte according to item 3, in which the concentration of the melamine compound and/or the polymer including the melamine compound as a monomer is 0.1 to 100 µmol/L.

Item 5. The electrolyte according to any one of items 1 to 4, in which the electrolyte is an electrolyte for a fuel cell or a metal-air battery.

Item 6. An oxidation inhibitor of an electrode electrochemical oxygen reduction catalyst including a melamine compound and/or a polymer including the melamine compound as a monomer.

Item 7. The oxidation inhibitor according to item 6, in which the melamine compound is represented by the general formula (1): 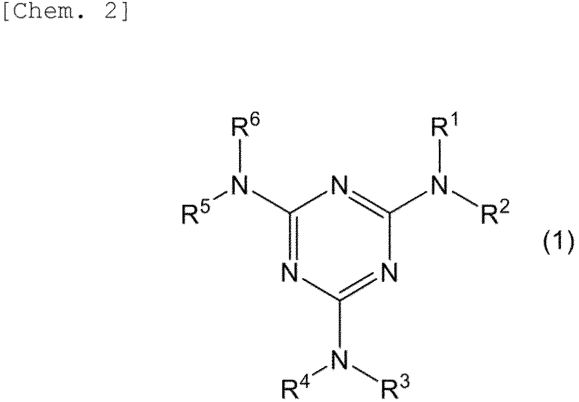 [in the formula, R¹, R², R³, R⁴, R⁵, and R⁶ are the same or different and each represents a hydrogen atom, a hydroxy group, a halogen atom, a substituted or unsubstituted alkyl group, or a substituted or unsubstituted alkenyl group].

Item 8. The oxidation inhibitor according to item 6 or 7, in which the oxidation inhibitor is the melamine compound and/or a solution including the melamine compound.

Item 9. The oxidation inhibitor according to item 8, in which the concentration of the melamine compound and/or the polymer including the melamine compound as a monomer is 0.1 to 100 µmol/L.

Item 10. The oxidation inhibitor according to any one of items 6 to 9, in which the electrode electrochemical oxygen reduction catalyst includes platinum nanoparticles.

Item 11. The oxidation inhibitor according to any one of items 6 to 10, in which the electrode electrochemical oxygen reduction catalyst is a cathode catalyst for a fuel cell.

Item 12. A fuel cell comprising the electrolyte according to any one of items 1 to 5 or the oxidation inhibitor according to any one of items 6 to 11.

Item 13. A metal-air battery including the electrolytic solution according to any one of items 1 to 5 or the oxidation inhibitor according to any one of items 6 to 11.

### Effects of the Invention

According to the present invention, it is possible to provide an electrolyte capable of efficiently suppressing a platinum catalyst from deteriorating in an air electrode.

### BRIEF DESCRIPTION OF THE DRAWINGS

FIG. 1 is a linear sweep voltammogram showing results of oxygen reduction activity before and after a durability test (0.6 V to 1.0 V, 10,000 cycles) of Example 1;
FIG. 2 is a linear sweep voltammogram showing results of oxygen reduction activity before and after a durability test (0.6 V to 1.0 V, 10,000 cycles) of Example 2;
FIG. 3 is a linear sweep voltammogram showing results of oxygen reduction activity before and after a durability test (0.6 V to 1.0 V, 10,000 cycles) of Example 3;
FIG. 4 is a linear sweep voltammogram showing results of oxygen reduction activity before and after a durability test (0.6 V to 1.0 V, 10,000 cycles) of Example 4;
FIG. 5 is a linear sweep voltammogram showing results of oxygen reduction activity before and after a durability test (0.6 V to 1.0 V, 10,000 cycles) of Comparative Example 1;
FIG. 6 is a graph showing changes in oxygen reduction activity before and after durability tests (0.6 V to 1.0 V, 10,000 cycles) of Examples 1, 2, 3, and 4 and Comparative Examples 1; FIG. 7 is a result of measuring average particle diameters of platinum catalysts of Example 1 and Comparative Example 1 after durability tests (0.6 V to 1.0 V, 10,000 cycles) by transmission electron microscope (TEM);

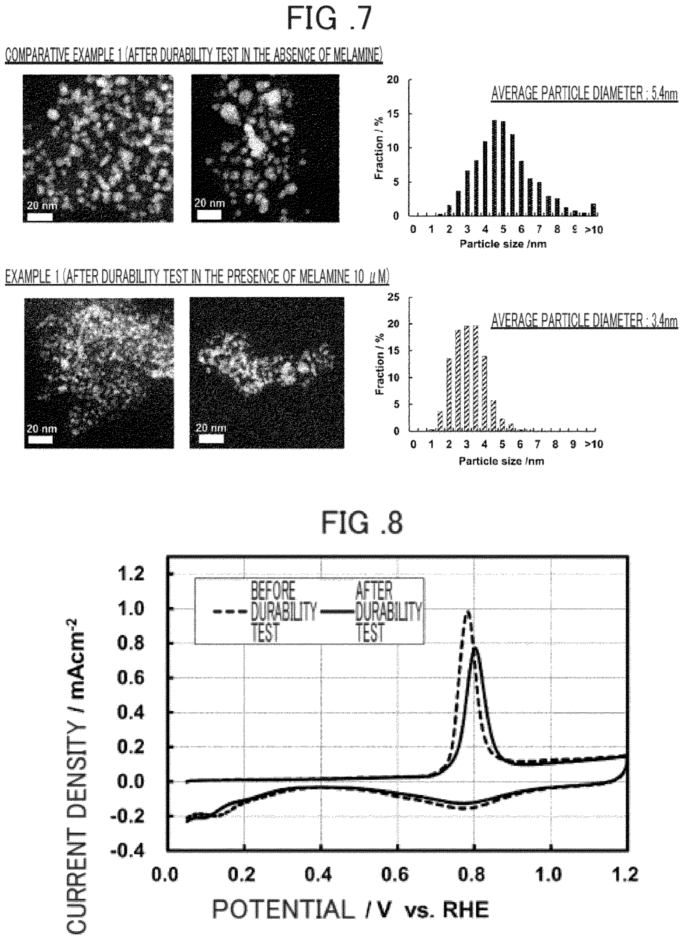

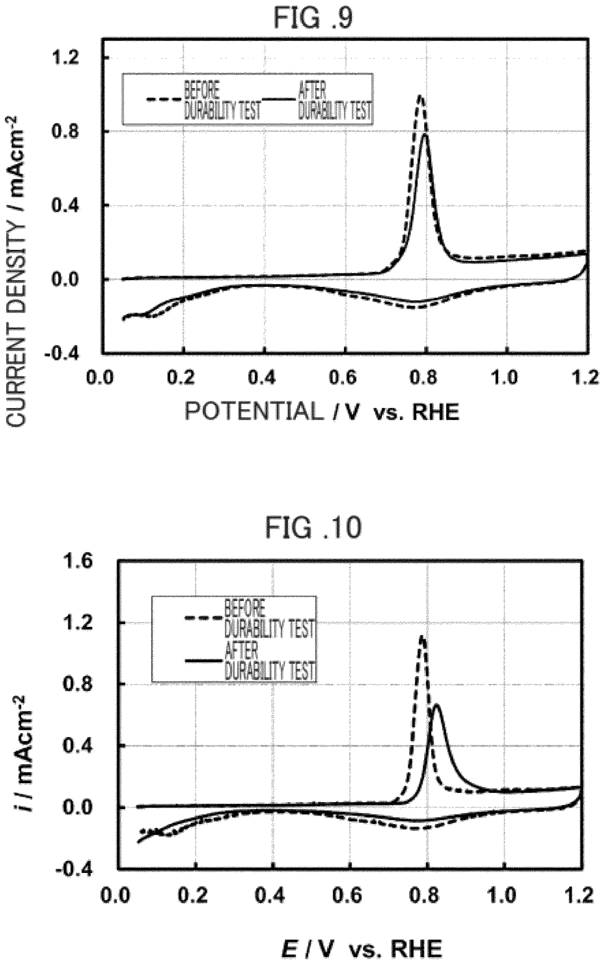

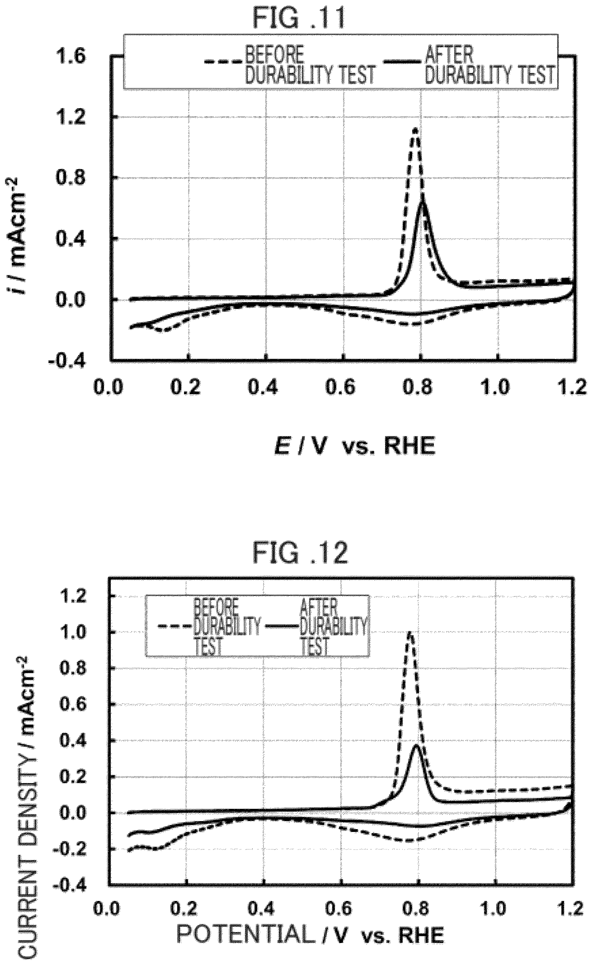

FIG. 8 is a CO stripping voltammogram before and after the durability test (0.6 V to 1.0 V, 10,000 cycles) of Example 1; FIG. 9 is a CO stripping voltammogram before and after the durability test (0.6 V to 1.0 V, 10,000 cycles) of Example 2; FIG. 10 is a CO stripping voltammogram before and after the durability test (0.6 V to 1.0 V, 10,000 cycles) of Example 3; FIG. 11 is a CO stripping voltammogram before and after the durability test (0.6 V to 1.0 V, 10,000 cycles) of Example 4; FIG. 12 is a CO stripping voltammogram before and after the durability test (0.6 V to 1.0V, 10,000 cycles) of Comparative

### Example 1; and

FIG. 13 is a graph showing peak area changes of CO stripping voltammograms before and after the durability tests (0.6 V to 1.0 V, 10,000 cycles) regarding platinum catalysts of Examples 1, 2, 3, and 4 and Comparative Example 1.

### PREFERRED MODE FOR CARRYING OUT THE INVENTION

In this specification, "comprising" is a concept including any of "consisting essentially" and "consisting of".

Further, in the present specification, when a numerical range is denoted by "A to B", "A or more and B or less" is indicated.

### 1. Electrolytes and Oxidation Inhibitors

The electrolyte of the present invention includes a melamine compound and/or a polymer including the melamine compound as a monomer. The electrolyte of the present invention may include the melamine compound and/or a polymer including the melamine compound as a monomer singly or in combination of two or more types thereof.

In the present invention, a melamine compound or a polymer thereof is blended with an electrolyte, whereby the electrolyte of the present invention does not function as a protective film of a platinum catalyst but suppresses oxidation of the platinum catalyst and can function as an oxidation inhibitor for an electrode electrochemical oxygen reduction catalyst, which increases the particle diameter of the platinum catalyst; as a result, the electrolyte can suppress the particle diameter of the platinum catalyst from increasing during charge and discharge, resulting in suppression of a surface area of the platinum catalyst from decreasing during charge and discharge, thereby improving durability of a fuel cell or a metal-air battery.

### (1-1) Melamine Compound and/or a Polymer including Melamine Compound as Monomer

As the melamine compound, not only melamine but also derivatives thereof can be used without any limitation. For example, a compound represented by the general formula (1): 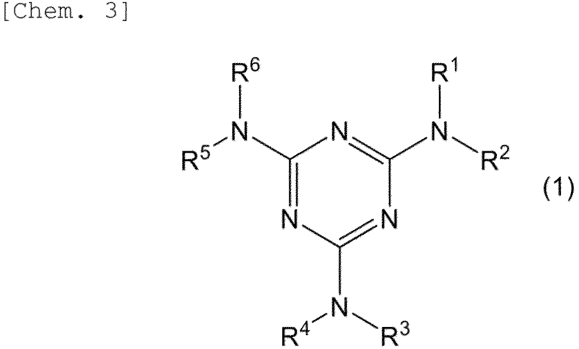 [in the formula, R¹, R², R³, R⁴, R⁵, and R⁶ are the same or different and each represents a hydrogen atom, a hydroxy group, a halogen atom, a substituted or unsubstituted alkyl group, or a substituted or unsubstituted alkenyl group].

In the general formula (1), examples of the halogen atom represented by R¹, R², R³, R⁴, R⁵, and R⁶ include a fluorine atom, a chlorine atom, a bromine atom, and an iodine atom.

In the general formula (1), examples of the alkyl group represented by R¹, R², R³, R⁴, R⁵ and R⁶ include lower alkyl groups, such as a methyl group, an ethyl group, a n-propyl group, an isopropyl group, a n-butyl group, an isobutyl group, a sec-butyl group, a tert-butyl group, a n-pentyl group, a n-hexyl group, a n-heptyl group and a n-octyl group (in particular, a linear or branched alkyl group having 1 to 10 carbon atoms and more preferably 1 to 8 carbon atoms). The alkyl group may also have 1 to 6 (in particular, 1 to 3) substituents, such as a hydroxy group and a halogen atom described above.

In the general formula (1), examples of the alkenyl group represented by R¹, R², R³, R⁴, R⁵, and R⁶ include lower alkenyl groups (in particular, linear or branched alkenyl groups having 2 to 10 carbon atoms and more preferably 2 to 8 carbon atoms) such as a vinyl group, an allyl group, a 2-butenyl group, a 3-butenyl group, a 1-methylallyl group, a 2-pentenyl group, and a 2-hexenyl group. This alkenyl group may have 1 to 6 (in particular, 1 to 3) substituents such as a hydroxy group and a halogen atom described above.

In the general formula (1), R¹, R², R³, R⁴, R⁵, and R⁶ are preferably a hydrogen atom, a substituted or unsubstituted alkyl group, or a substituted or unsubstituted alkenyl group and more preferably a hydrogen atom or a substituted or unsubstituted alkyl group, from the viewpoints of being easy to suppress oxidation of a platinum catalyst, being easy to improve durability of a fuel cell or a metal-air battery, and being easy to perform oxygen reduction reaction at a higher potential and further improve oxygen reduction activity (being easy to decrease overvoltage). Note that R¹, R², R³, R⁴, R⁵, and R⁶ may be the same or different.

Examples of the melamine compound satisfying the above conditions include the following: 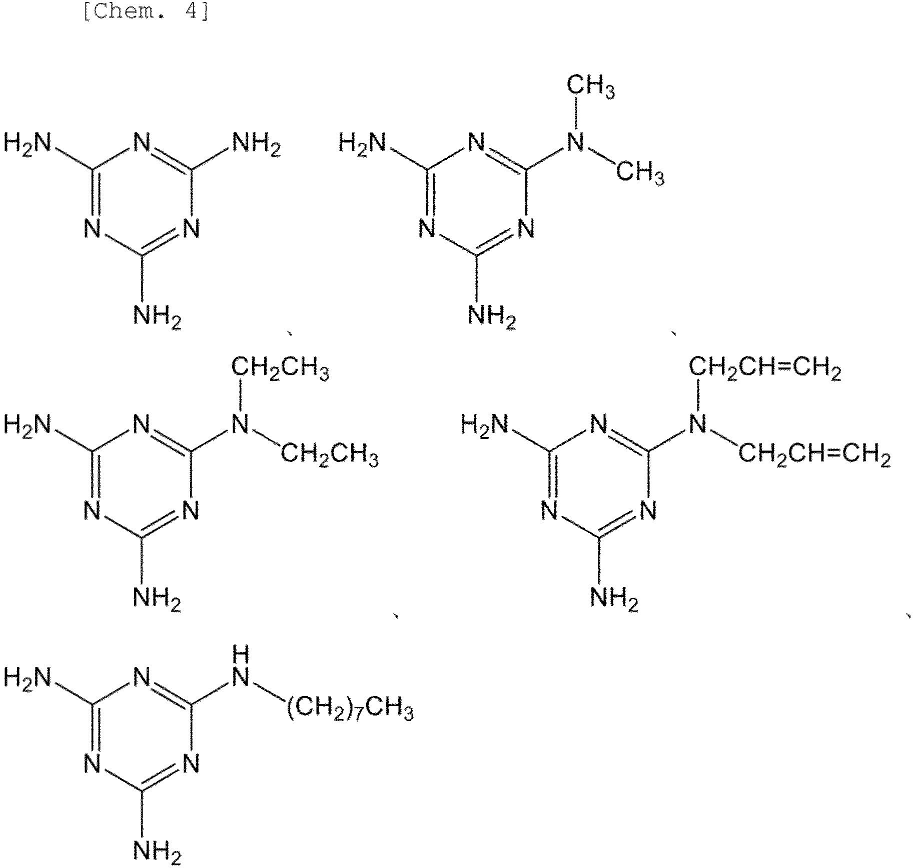 and the like. Such a melamine compound may be a commercially available product or may be independently synthesized.

As the polymer including the melamine compound as a monomer, a melamine resin having the melamine compound described above in a main chain of a repeating unit can be used. For example, a melamine resin or the like having a repeating unit represented by a general formula (2) may be used: 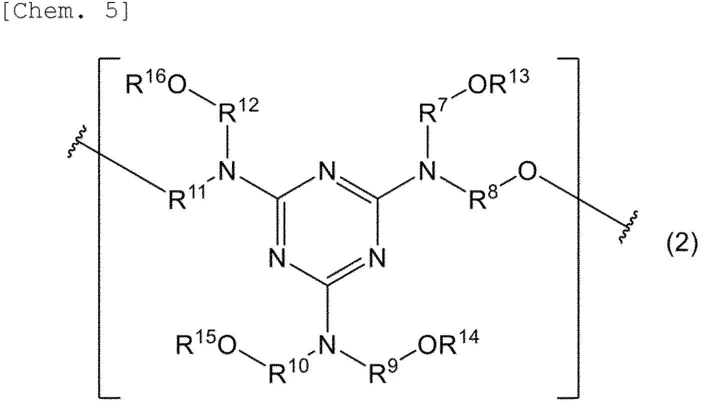 [in the formula, R⁷, R⁸, R⁹, R¹⁰, R¹¹, and R¹² are the same or different, and each represents a divalent group;
R¹³, R¹⁴, R¹⁵, and R¹⁶ are the same or different and each represents a hydrogen atom, a substituted or unsubstituted alkyl group, or a substituted or unsubstituted alkenyl group;
OR¹³, OR¹⁴, OR¹⁵, and OR¹⁶ may be partially or entirely eliminated and crosslinked with a divalent group represented by R⁷, R⁸, R⁹, R¹⁰, R¹¹, and R¹² to form a three-dimensional network structure] .

Note that use of a resin having no melamine skeleton in the main chain and having a melamine skeleton in the side chain does not make it possible to improve durability of a fuel cell or a metal-air battery or to obtain an effect of improving oxygen reduction activity, because such a resin has no effect of suppressing oxidation of a platinum catalyst.

Examples of the divalent group represented by R⁷, R⁸, R⁹, R¹⁰, R¹¹, and R¹² include alkylene groups and alkenylene groups.

Examples of the alkylene group include lower alkylene groups such as a methylene group, an ethylene group, a trimethylene group, and a propylene group (in particular, a linear or branched alkylene group having 1 to 10 carbon atoms and more preferably 1 to 8 carbon atoms). The alkylene group may also have 1 to 6 (in particular 1 to 3) substituents such as a hydroxy group and a halogen atom described above.

Examples of the alkenylene group include lower alkenylene groups, such as a vinylene group, an allylene group, and a butenylene group (in particular, an alkenylene group having 2 to 10 carbon atoms and more preferably 2 to 8 carbon atoms). The alkenylene group may have 1 to 6 (in particular, 1 to 3) substituents such as a hydroxy group and a halogen atom described above.

As R⁷, R⁸, R⁹, R¹⁰, R¹¹, and R¹² in the general formula (2), the substituted or unsubstituted alkylene group is preferable from the viewpoints of being easy to suppress oxidation of a platinum catalyst, being easy to improve durability of a fuel cell or a metal-air battery, and being easy to perform oxygen reduction reaction at a higher potential to further improve oxygen reduction activity (being easy to decrease overvoltage) . Note that R⁷, R⁸, R⁹, R¹⁰, R¹¹, and R¹² may be the same or different.

As the alkyl group and the alkenyl group represented by R¹³, R¹⁴, R¹⁵ and R¹⁶ in the general formula (2), those described above can be adopted. The same applies to the type and number of substituents.

Examples of the repeating unit satisfying the above conditions include the following: 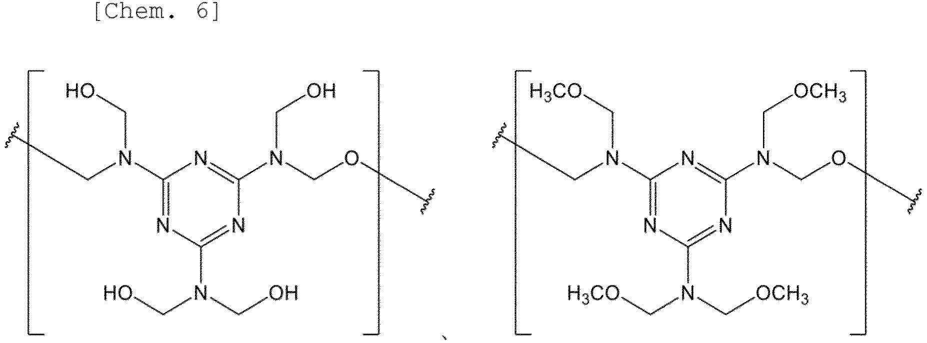 or the like.

The polymer including such a melamine compound as a monomer may be a polymer of a melamine resin represented by a general formula (3): 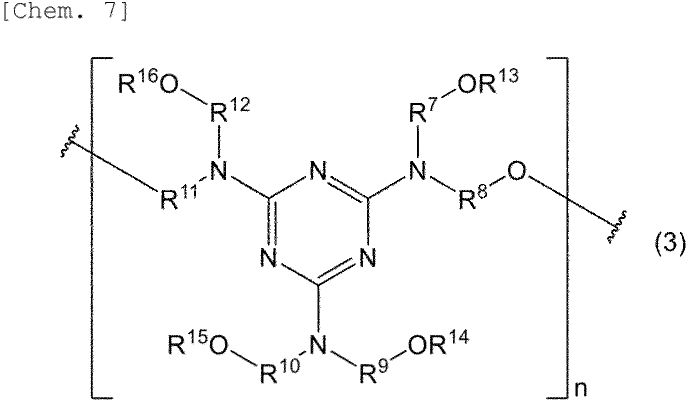 [in the formula, R⁷, R⁸, R⁹, R¹⁰, R¹¹, R¹², R¹³, R¹⁴, R¹⁵ and R¹⁶ are the same as defined above, and
n represents an integer of 2 to 1,000],
as a polymer consisting of a repeating unit represented by the general formula (2).

The polymer including the melamine compound as a monomer can also include a repeating unit other than the repeating unit represented by the general formula (2) (for example, a structure in which OR¹³, OR¹⁴, OR¹⁵, and OR¹⁶ are partially or entirely eliminated, followed by crosslinking with a divalent group (alkylene group, alkenylene group, etc.) represented by R⁷, R⁸, R⁹, R¹⁰, R¹¹, and R¹² to form a three-dimensional network structure).

A degree of polymerization (corresponding to n in the case of the melamine resin represented by the general formula (3)) of the polymer including the melamine compound as a monomer is not particularly limited, and an average (typical) degree of polymerization degree is preferably 2 to 1,000 and more preferably 3 to 500, from the viewpoints of being easy to suppress oxidation of a platinum catalyst, being easy to improve durability of a fuel cell or a metal-air battery, and being easy to perform oxygen reduction reaction at a higher potential to further improve oxygen reduction activity (being easy to decrease overvoltage).

Note that a terminal group of the polymer including the melamine compound as a monomer is not particularly limited. Usually, the terminal group may be a hydrogen atom, a hydroxy group, the alkyl groups described above, the alkenyl groups described above, or the like.

### (1-2) Electrolyte

The electrolyte of the present invention may be a solid electrolyte film when it is used as an electrolyte applied to a fuel cell using a solid electrolyte such as a polymer electrolyte fuel cell (PEFC).

When the electrolyte of the present invention is used in the form of a solid electrolyte film, the solid electrolyte film includes a solid electrolyte such as various types of ion exchange resins such as a perfluorosulfonic acid polymer, an alkylammonium polymer, or the like, and can be formed into a film by a conventional method. A melamine-including solid electrolyte can be prepared by introducing melamine into this molded electrode by ion exchange or impregnation with a melamine solution.

When the electrolyte of the present invention is formed into a solid electrolyte film, the content of the melamine compound and/or the polymer including the melamine compound as a monomer is not particularly limited. For example, from the viewpoints of being easy to suppress oxidation of a platinum catalyst, being easy to improve durability of a fuel cell or a metal-air battery, and being easy to perform oxygen reduction reaction at a higher potential to further improve oxygen reduction activity (being easy to decrease overvoltage), the content of the melamine compound and/or the polymer including the melamine compound as a monomer is preferably 0.000001 to 0.002 parts by mass and more preferably 0.000005 to 0.0002 parts by mass, per 100 parts by mass of the solid electrolyte film. Note that when a plurality of melamine compounds and/or polymers including a melamine compound as a monomer are used, the total amount thereof is preferably adjusted to fall within the above range. Note that this numerical range is a numerical range according to the results of the electrolytic solution in Examples to be described later.

On the other hand, when the electrolyte of the present invention is applied to batteries using, as the electrolytic solution, an aqueous solution, such as an alkaline fuel cell (AFC), a phosphoric acid fuel cell (PAFC), or a metal-air battery, the electrolyte can be an electrolytic solution in the form of a solution.

When the electrolyte of the present invention is used as an electrolytic solution, the electrolytic solution may be an aqueous solution including an electrolyte, such as potassium hydroxide, phosphoric acid, perchloric acid, sulfuric acid, or nitric acid, in addition to the melamine compound and/or the polymer including a melamine compound as a monomer.

When the electrolyte of the present invention is used as an electrolytic solution, the concentration of the melamine compound and/or the polymer including the melamine compound as a monomer is not particularly limited. For example, the concentration of the melamine compound and/or the polymer including the melamine compound as a monomer is preferably 0.1 to 100 µmol/L and more preferably 0.5 to 20 pmol/L, from the viewpoints of being easy to suppress oxidation of a platinum catalyst, being easy to improve durability of a fuel cell or a metal-air battery, and being easy to perform oxygen reduction reaction at a higher potential to further improve oxygen reduction activity (being easy to decrease overvoltage). Above all, in the case of using a melamine compound in which at least one of R¹, R², R³, R⁴, R⁵, and/or R⁶ in the general formula (1) is other than a hydrogen atom (a hydroxy group, a halogen atom, a substituted or unsubstituted alkyl group, or a substituted or unsubstituted alkenyl group) or a polymer including such a melamine compound as a monomer, it is possible to adjust the concentration of the melamine compound and/or the polymer including the melamine compound as a monomer to a high concentration, for example, 2 to 100 µmol/L and preferably 5 to 20 µmol/L, from the viewpoints of being easy to suppress oxidation of a platinum catalyst, being easy to improve durability of a fuel cell or a metal-air battery, and being easy to perform oxygen reduction reaction at a higher potential to further improve oxygen reduction activity (being easy to decrease overvoltage). Similarly, the concentration of the electrolyte is preferably 0.1 to 10 mol/L and more preferably 1 to 10 mol/L. When a plurality of melamine compounds and/or polymers including a melamine compound as a monomer are used, the total amount thereof is preferably adjusted to fall within the above range.

The electrolyte of the present invention as described above does not function as a protective film of a platinum catalyst but suppresses oxidation of the platinum catalyst and can function as an oxidation inhibitor for an electrode electrochemical oxygen reduction catalyst, which increases the particle diameter of the platinum catalyst; as a result, the electrolyte can suppress the particle diameter of the platinum catalyst from increasing during charge and discharge, resulting in suppression of a decrease in the surface area of the platinum catalyst, thereby improving durability of a fuel cell or a metal-air battery. For this reason, the electrolyte of the present invention is useful as an electrolyte for a fuel cell (in particular, an electrolyte film of a fuel cell or an electrolyte for an electrolytic solution) or an electrolyte for a metal-air battery (in particular, an electrolyte for an electrolytic solution of a metal-air battery).

### 2. Battery

When the battery of the present invention is a polymer electrolyte fuel cell (PEFC) using a solid electrolyte, the solid electrolyte film includes a solid electrolyte including various types of ion exchange resins such as a perfluorosulfonic acid polymer and an alkyl ammonium polymer and can be formed into a film by a conventional method. A melamine-including solid electrolyte can be prepared by introducing melamine into this molded electrode by ion exchange or impregnation with a melamine solution.

When the battery of the present invention is a battery using an aqueous solution as the electrolytic solution, such as an alkaline fuel cell (AFC) using an electrolytic solution, a phosphoric acid fuel cell (PAFC), or a metal-air battery, the electrolytic solution may be an aqueous solution including an electrolyte such as potassium hydroxide, phosphoric acid, perchloric acid, sulfuric acid, or nitric acid, in addition to the melamine compound and/or the polymer including a melamine compound as a monomer, as described above.

In the battery of the present invention, the configuration other than the electrolyte film or the electrolytic solution may be the same as that of a fuel cell or a metal-air battery conventionally used.

For example, an air electrode in the battery of the present invention may have an air electrode catalyst layer using an electrochemical oxygen reduction catalyst such as conventionally known platinum nanoparticles. As the electrochemical oxygen reduction catalyst which can be used, for example, the electrochemical oxygen reduction catalysts described in Japanese Unexamined Patent Application, Publication Nos. 2017-010853 and 2018-086640 and Internation Publication Nos. WO2019/019229 and WO2021/090746 can be mentioned.

The thickness of the air electrode catalyst layer is not particularly limited, and may be usually about 0.1 to 100 um. The amount of the catalyst is not particularly limited, and may be, for example, about 0.01 to 20 mg/cm².

A method for forming such an air electrode catalyst layer is not particularly limited, and the air electrode catalyst layer may be formed by a method of applying a catalyst ink prepared by mixing an electrochemical oxygen reduction catalyst and a resin solution to a gas diffusion layer, a current collector, or the like, followed by drying.

Other configurations of the air electrode may be the same as those of a well-known air electrode. For example, the air electrode may have a structure in which a current collecting material such as carbon paper, carbon cloth, metal mesh, a metal sintered body, a foamed metal plate, a metal porous body, or the like is disposed on a catalyst layer side of the air electrode and further, a water-repellent film, a diffusion film, an air distribution layer, or the like is disposed.

The structure of the fuel electrode is not particularly limited, and may be the same as that of a well-known battery. As a catalyst for a fuel electrode, conventionally known various metals, metal alloys, metal complexes, or the like can be used. Examples of metal species that can be used include noble metals such as platinum, palladium, iridium, rhodium, ruthenium, gold, or the like and additionally, base metals such as nickel, silver, cobalt, iron, copper, zinc or the like. A single metal catalyst selected from these metals, a metal complex, an alloy consisting of any combination of two or more metals, or a composite of a metal complex may be used. The catalyst can also be used as a composite catalyst formed of a metal catalyst selected from the above and another metal oxide or as a supported catalyst in which catalyst fine particles are dispersed on a carrier such as a carbonaceous material or a metal oxide.

In the case of manufacturing a polymer electrolyte fuel cell, the polymer electrolyte fuel cell can be manufactured by sandwiching both surfaces of a film-electrode assembly obtained by using an electrolyte film with a current collector such as carbon paper or carbon cloth and incorporating it into a cell.

On the other hand, when the present invention is applied not to a polymer electrolyte fuel cell but to a battery using an aqueous electrolytic solution such as a phosphoric acid fuel cell or the like, the separator may be impregnated with the electrolytic solution. Other members are the same as those of the polymer electrolyte fuel cell.

When the battery of the present invention is used in a metal-air battery, the positive electrode of the metal-air battery can be the same as the above-described air electrode. As a metal negative electrode of the metal-air battery, a metal such as zinc, aluminum, magnesium, or iron can be used. The specific structure of the metal negative electrode can be the same as that of a known metal-air battery. The other members are the same as those of the polymer electrolyte fuel cell.

In the battery having the above structure, oxygen or air can be supplied or naturally diffused to the air electrode side in any case. Further, a substance serving as a fuel can be supplied to the fuel electrode side of a fuel cell (in particular, a polymer electrolyte fuel cell, a phosphoric acid fuel cell, or the like). As the fuel substance, in addition to hydrogen gas, alcohols such as methanol, ethanol, isopropanol, and ethylene glycol, formic acid, borohydride salts, hydrazine, solutions of sugars, and the like can be used.

When the battery of the present invention is a fuel cell (in particular, a polymer electrolyte fuel cell or a phosphoric acid fuel cell), the operation temperature varies depending on the electrolyte used, but is usually about 0 to 250°C and preferably about 10 to 100°C.

### EXAMPLES

Hereinafter, the present invention will be described in more detail with reference to Examples and Comparative Examples. The present invention is not limited to the following Examples.

As the melamine, commercially available products (manufactured by Tokyo Chemical Industry) were used.

### Production Example 1: Synthesis of Octylmelamine

Equimolar amounts of 2-chloro-4,6-diamino-1,3,5-triazine (manufactured by Tokyo Chemical Industry) and n-octylamine (manufactured by Tokyo Chemical Industry) were added to a mixed solvent of water and dioxane and the mixture was refluxed while stirring. Sodium hydroxide in an equimolar amount with respect to that of the raw material was dissolved in water in advance, slowly added to the reaction solution, and further refluxed.

The reaction product was separated with a mixed solvent of water and toluene and the toluene layer was collected to obtain a crude product. The crude product was purified by flash column chromatography, and then the production of octylmelamine as a target product was confirmed by NMR.
¹H NMR (CDCl₃, 600 MHz)
δ 0.88 (3H, t, J=7 Hz, -CH₃), 1.28-1.55 (12H, m, -CH₂-), 3.31-3.34 (2H, m, -CH₂-), 4.88-5.01 (5H, m, -NH₂, -NH-)

The structural formula of octylmelamine is as follows:

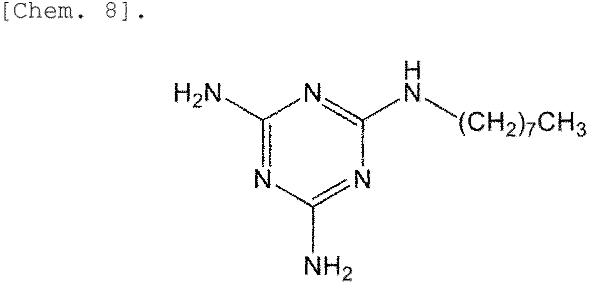

### Example 1

5 mg of a platinum catalyst (TEC10E50E manufactured by Tanaka Kikinzoku Kogyo K.K.; average particle size: 2.5 nm) was suspended in a mixed solvent of 5.14 mL of ultrapure water and 1.62 mL of 2-propanol and 3.6 µL of this suspension was added dropwise onto a glassy carbon electrode (surface area: 0.0707 cm²) manufactured by B. A. S, Ltd. and dried to obtain a catalyst-modified electrode.

Electrochemical measurement was performed by a three-electrode method at 25°C, by using the obtained catalyst-modified electrode as a working electrode, a reversible hydrogen electrode as a reference electrode, and a platinum coil as a counter electrode. As an electrolytic solution, a 0.1 mol/L aqueous solution of perchloric acid was used. First, a cyclic voltammogram was measured under an argon atmosphere, then the gas atmosphere was changed to oxygen, and a linear sweep voltammogram was measured from a low potential side. Thus, oxygen reduction activity before a durability test when the electrolytic solution includes melamine was evaluated.

Further, a CO stripping voltammogram was measured to evaluate a change in platinum surface areas. The potential was held at 0.05 V for 600 seconds under a CO atmosphere at 25°C and then, a cyclic voltammogram was measured under an argon atmosphere. The change in platinum surface areas before and after the durability test can be evaluated by this measurement, because the peak area of the cyclic voltammogram after CO adsorption is proportional to the platinum surface area.

Next, 10 µmol/L of melamine was dissolved in an electrolytic solution, a square wave potential cycle of 10,000 cycles was applied between 0.6 V (holding for 3 seconds) and 1.0 V (holding for 3 seconds) at 79°C under an argon atmosphere ("Proposals of Goals, Research and Development Tasks, and Evaluation Methods for Polymer Electrolyte Fuel Cells" by Fuel Cell Commercialization Conference of Japan). Then, the cyclic voltammogram and a linear sweep voltammogram were measured again in the same manner. Thereby, a retention ratio (durability) of the oxygen reduction activity of the platinum catalyst when the electrolytic solution includes melamine can be evaluated. Further, a CO stripping voltammogram was measured and the peak area was evaluated. By comparing the obtained peak area with the peak area of the CO stripping voltammogram before the durability test, a change in platinum surface areas after the durability test when the electrolytic solution included melamine was evaluated.

### Example 2

5 mg of a platinum catalyst (TEC10E50E manufactured by Tanaka Kikinzoku Kogyo K.K.; average particle size: 2.5 nm) was suspended in a mixed solvent of 5.14 mL of ultrapure water and 1.62 mL of 2-propanol and 3.6 µL of this suspension was added dropwise onto a glassy carbon electrode (surface area: 0.0707 cm²) manufactured by B. A. S, Ltd. and dried to obtain a catalyst-modified electrode.

Electrochemical measurement was performed by a three-electrode method at 25°C, by using the obtained catalyst-modified electrode as a working electrode, a reversible hydrogen electrode as a reference electrode, and a platinum coil as a counter electrode. As an electrolytic solution, a 0.1 mol/L aqueous solution of perchloric acid was used. First, a cyclic voltammogram was measured under an argon atmosphere, then the gas atmosphere was changed to oxygen, and a linear sweep voltammogram was measured from a low potential side. Thus, oxygen reduction activity before a durability test when the electrolytic solution includes melamine was evaluated.

Further, a CO stripping voltammogram was measured to evaluate a change in platinum surface areas. The potential was held at 0.05 V for 600 seconds under a CO atmosphere at 25°C and then, a cyclic voltammogram was measured under an argon atmosphere. The change in platinum surface areas before and after the durability test can be evaluated by this measurement, because the peak area of the cyclic voltammogram after CO adsorption is proportional to the platinum surface area.

Next, 1 µmol/L of melamine was dissolved in an electrolytic solution, a square wave potential cycle of 10,000 cycles was applied between 0.6 V (holding for 3 seconds) and 1.0 V (holding for 3 seconds) at 79°C under an argon atmosphere ("Proposals of Goals, Research and Development Tasks, and Evaluation Methods for Polymer Electrolyte Fuel Cells" by Fuel Cell Commercialization Conference of Japan). Then, the cyclic voltammogram and a linear sweep voltammogram were measured again in the same manner. Thereby, a retention ratio (durability) of the oxygen reduction activity of the platinum catalyst when the electrolytic solution includes melamine can be evaluated. Further, a CO stripping voltammogram was measured and the peak area was evaluated. By comparing the obtained peak area with the peak area of the CO stripping voltammogram before the durability test, a change in platinum surface areas after the durability test when the electrolytic solution included melamine was evaluated.

### Example 3

5 mg of a platinum catalyst (TEC10E50E manufactured by Tanaka Kikinzoku Kogyo K.K.; average particle size: 2.5 nm) was suspended in a mixed solvent of 5.14 mL of ultrapure water and 1.62 mL of 2-propanol and 3.6 µL of this suspension was added dropwise onto a glassy carbon electrode (surface area: 0.0707 cm²) manufactured by B. A. S, Ltd. and dried to obtain a catalyst-modified electrode.

Electrochemical measurement was performed by a three-electrode method at 25°C, by using the obtained catalyst-modified electrode as a working electrode, a reversible hydrogen electrode as a reference electrode, and a platinum coil as a counter electrode. As an electrolytic solution, a 0.1 mol/L aqueous solution of perchloric acid was used. First, a cyclic voltammogram was measured under an argon atmosphere, then the gas atmosphere was changed to oxygen, and a linear sweep voltammogram was measured from a low potential side. Thus, the oxygen reduction activity before a durability test when the electrolytic solution includes melamine was evaluated.

Further, a CO stripping voltammogram was measured to evaluate a change in platinum surface areas. The potential was held at 0.05 V for 600 seconds under a CO atmosphere at 25°C and then, a cyclic voltammogram was measured under an argon atmosphere. The change in platinum surface areas before and after the durability test can be evaluated by this measurement, because the peak area of the cyclic voltammogram after CO adsorption is proportional to the platinum surface area.

Next, 10 µmol/L of octylmelamine was dissolved in an electrolytic solution, a square wave potential cycle of 10,000 cycles was applied between 0.6 V (holding for 3 seconds) and 1.0 V (holding for 3 seconds) at 79°C under an argon atmosphere ("Proposals of Goals, Research and Development Tasks, and Evaluation Methods for Polymer Electrolyte Fuel Cells" by Fuel Cell Commercialization Conference of Japan). Then, the cyclic voltammogram and a linear sweep voltammogram were measured again in the same manner. Thereby, a retention ratio (durability) of the oxygen reduction activity of the platinum catalyst when the electrolytic solution includes octylmelamine can be evaluated. Further, a CO stripping voltammogram was measured and the peak area was evaluated. By comparing the obtained peak area with the peak area of the CO stripping voltammogram before the durability test, a change in platinum surface areas after the durability test when the electrolytic solution included octylmelamine was evaluated.

### Example 4

5 mg of a platinum catalyst (TEC10E50E manufactured by Tanaka Kikinzoku Kogyo K.K.; average particle size: 2.5 nm) was suspended in a mixed solvent of 5.14 mL of ultrapure water and 1.62 mL of 2-propanol and 3.6 µL of this suspension was added dropwise onto a glassy carbon electrode (surface area: 0.0707 cm²) manufactured by B. A. S, Ltd. and dried to obtain a catalyst-modified electrode.

Electrochemical measurement was performed by a three-electrode method at 25°C, by using the obtained catalyst-modified electrode as a working electrode, a reversible hydrogen electrode as a reference electrode, and a platinum coil as a counter electrode. As an electrolytic solution, a 0.1 mol/L aqueous solution of perchloric acid was used. First, a cyclic voltammogram was measured under an argon atmosphere, then the gas atmosphere was changed to oxygen, and a linear sweep voltammogram was measured from a low potential side. Thus, oxygen reduction activity before a durability test when the electrolytic solution includes melamine was evaluated.

Further, a CO stripping voltammogram was measured to evaluate a change in platinum surface areas. The potential was held at 0.05 V for 600 seconds under a CO atmosphere at 25°C and then, a cyclic voltammogram was measured under an argon atmosphere. The change in platinum surface areas before and after the durability test can be evaluated by this measurement, because the peak area of the cyclic voltammogram after CO adsorption is proportional to the platinum surface area.

Next, 1 µmol/L of octylmelamine was dissolved in an electrolytic solution, a square wave potential cycle of 10,000 cycles was applied between 0.6 V (holding for 3 seconds) and 1.0 V (holding for 3 seconds) at 79°C under an argon atmosphere ("Proposals of Goals, Research and Development Tasks, and Evaluation Methods for Polymer Electrolyte Fuel Cells" by Fuel Cell Commercialization Conference of Japan). Then, the cyclic voltammogram and a linear sweep voltammogram were measured again in the same manner. Thereby, a retention ratio (durability) of the oxygen reduction activity of the platinum catalyst when the electrolytic solution includes octylmelamine can be evaluated. Further, a CO stripping voltammogram was measured and the peak area was evaluated. By comparing the obtained peak area with the peak area of the CO stripping voltammogram before the durability test, a change in platinum surface areas after the durability test when the electrolytic solution included octylmelamine was evaluated.

### Comparative Example 1

5 mg of a platinum catalyst (TEC10E50E manufactured by Tanaka Kikinzoku Kogyo K.K.; average particle size: 2.5 nm) was suspended in a mixed solvent of 5.14 mL of ultrapure water and 1.62 mL of 2-propanol and 3.6 µL of this suspension was added dropwise onto a glassy carbon electrode (surface area: 0.0707 cm²) manufactured by B. A. S, Ltd. and dried to obtain a catalyst-modified electrode.

Next, 0.1 mol/L of an aqueous perchloric acid solution was used as the electrolytic solution.

Electrochemical measurement was performed by a three-electrode method at 25°C, by using the obtained electrolytic solution, the obtained catalyst-modified electrode as a working electrode, a reversible hydrogen electrode as a reference electrode, and a platinum coil as a counter electrode. First, a cyclic voltammogram was measured under an argon atmosphere, then the gas atmosphere was changed to oxygen, and a linear sweep voltammogram was measured from a low potential side. Thus, the oxygen reduction activity during the durability test when the electrolytic solution included neither the melamine compound nor the polymer including the melamine compound as a monomer was evaluated.

Further, a CO stripping voltammogram was measured to evaluate a change in platinum surface areas. The potential was held at 0.05 V for 600 seconds under a CO atmosphere at 25°C and then, a cyclic voltammogram was measured under an argon atmosphere. The change in platinum surface areas before and after the durability test can be evaluated by this measurement, because the peak area of the cyclic voltammogram after CO adsorption is proportional to the platinum surface area.

Next, a square wave potential cycle of 10,000 cycles was applied between 0.6 V (holding for 3 seconds) and 1.0 V (holding for 3 seconds) at 79°C under an argon atmosphere ("Proposals of Goals, Research and Development Tasks, and Evaluation Methods for Polymer Electrolyte Fuel Cells" by Fuel Cell Commercialization Conference of Japan). Then, the cyclic voltammogram and a linear sweep voltammogram were measured again in the same manner. Thereby, a retention ratio (durability) of the oxygen reduction activity of the platinum catalyst when the electrolytic solution includes melamine can be evaluated. Further, a CO stripping voltammogram was measured and the peak area was evaluated. By comparing the obtained peak area with the peak area of the CO stripping voltammogram before the durability test, a change in platinum surface areas after the durability test when the electrolytic solution included melamine was evaluated.

### Results (Durability)

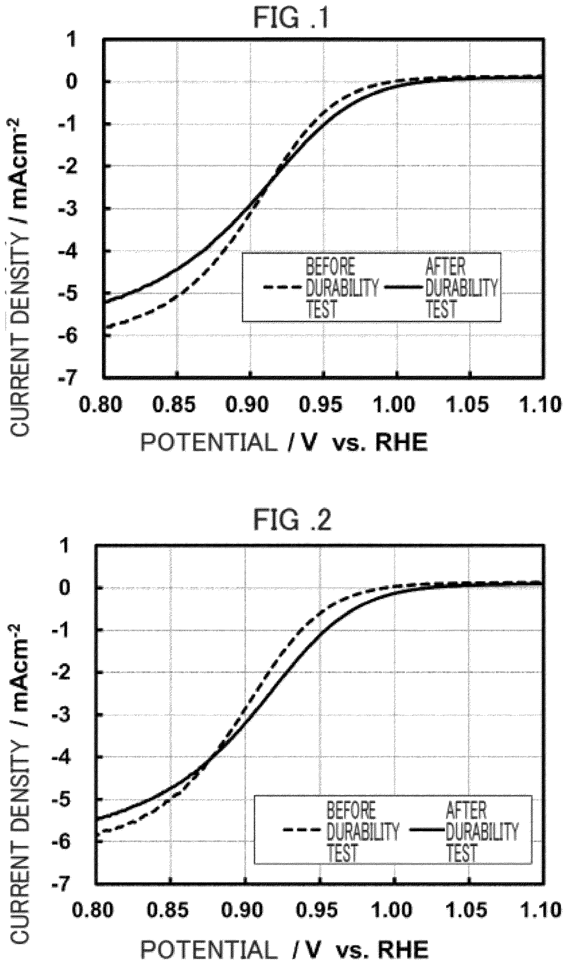

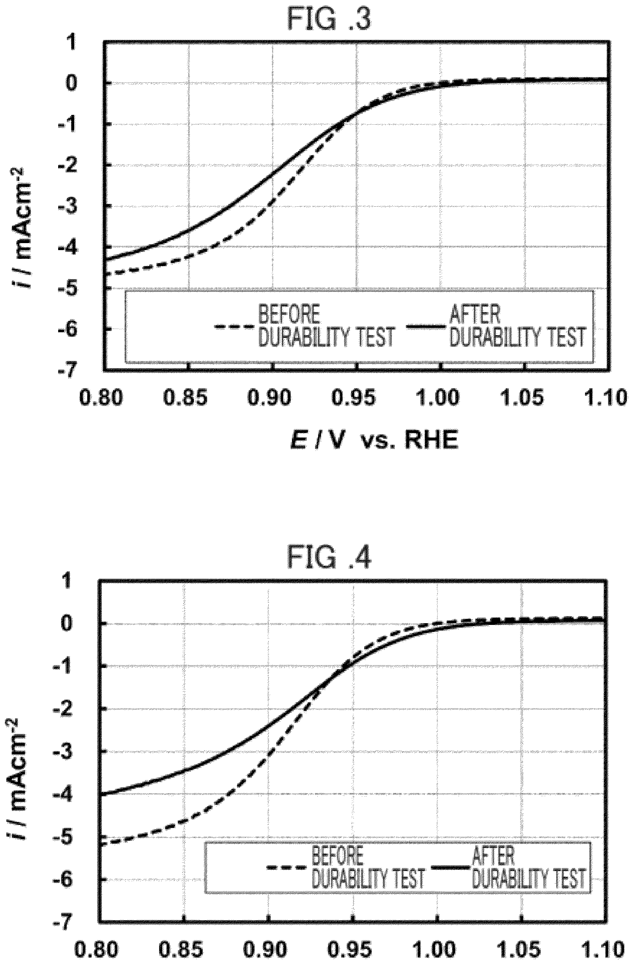

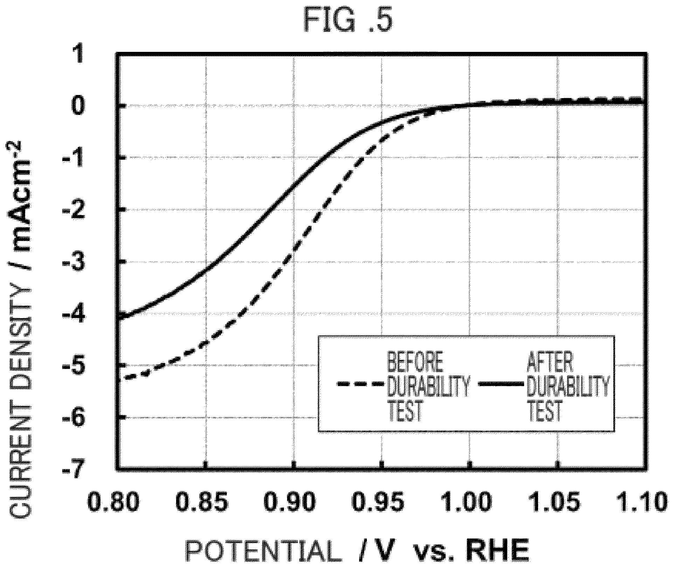

FIGs. 1 to 5 show the results of the oxygen reduction activity before and after the durability tests of Examples 1, 2, 3, and 4, and Comparative Example 1.

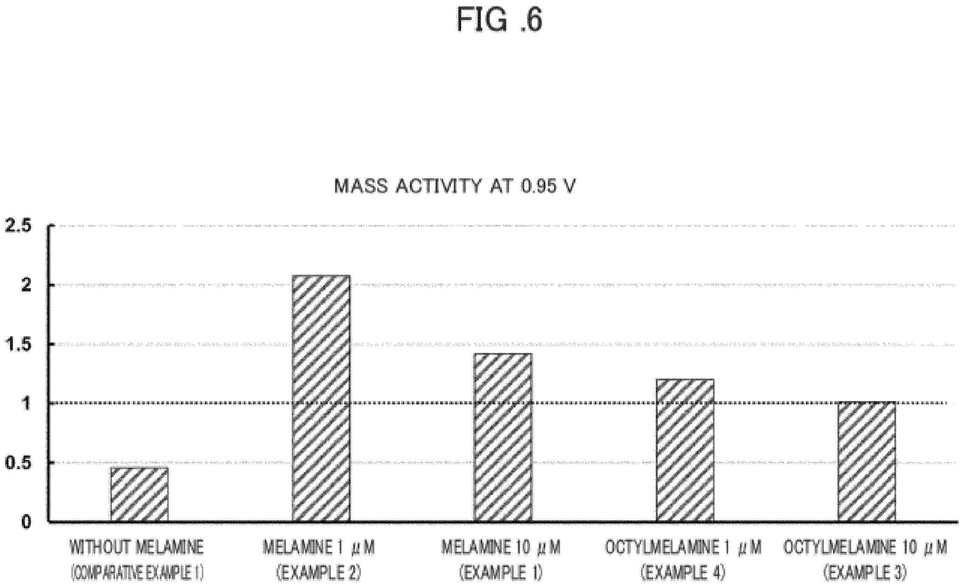

In Comparative Example 1, when the durability test of 10,000 cycles was performed between 0.6 V and 1.0 V, the electrochemical oxygen reduction activity per mass at 0.95 V was reduced by 54% after the durability test. Contrary to this, in Example 1, the electrochemical oxygen reduction activity per mass at 0.95 V increased by 42% after the durability test; in Example 2, the electrochemical oxygen reduction activity per mass at 0.95 V, on the contrary, increased by 108% after the durability test; in Example 3, the electrochemical oxygen reduction activity per mass at 0.95 V hardly changed before and after the durability test; and in Example 4, the electrochemical oxygen reduction activity per mass at 0.95 V, on the contrary, increased by 20% after the durability test. As a result, the change in oxygen reduction activity per mass before and after the durability test (oxygen reduction activity per mass after the durability test/oxygen reduction activity per mass before the durability test) was as shown in FIG. 6.

The average particle diameters of the platinum catalysts of Example 1 and Comparative Example 1 after the durability tests were measured by transmission electron microscope (TEM). The results are shown in FIG. 7.

In Comparative Example 1, the average diameter was about 5.4 nm. Perhaps because the oxidation of the platinum catalyst was not suppressed, platinum nanoparticles in the platinum catalyst grew bigger and a decrease in the active surface area was unavoidable, suggesting that the durability was degraded. On the other hand, in Example 1, the average diameter was about 3.4 nm. Perhaps because oxidation of the platinum catalyst was suppressed, platinum nanoparticles in the platinum catalyst did not grow bigger and decrease in the active surface area was suppressed, suggesting that the durability was not degraded.

FIGs. 8 to 12 show CO stripping voltammograms of the platinum catalysts of Examples 1, 2, 3, and 4 and Comparative Example 1 before and after the durability tests.

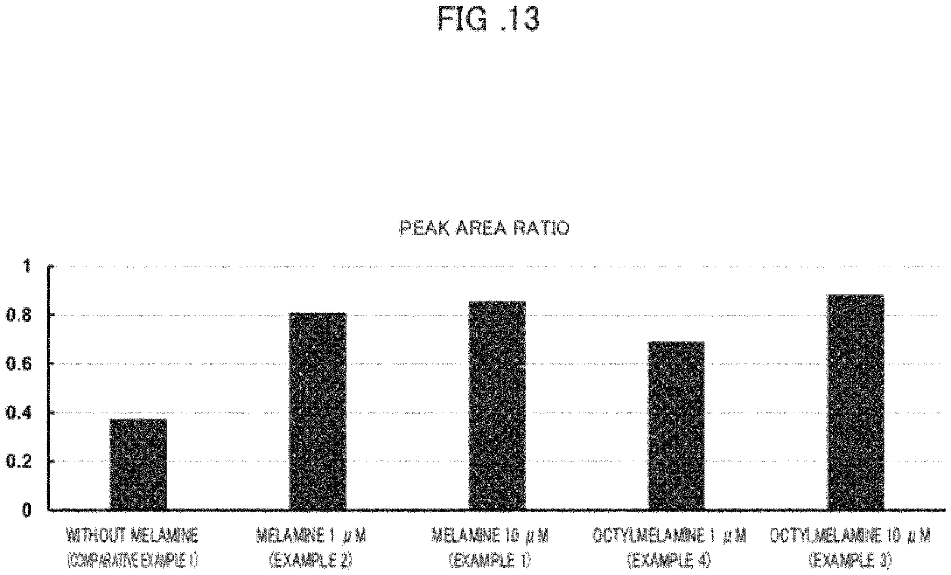

When the durability test of 10,000 cycles was performed between 0.6 V and 1.0 V, in Comparative Example 1, the integral value of the peak appearing near 0.8 V vs RHE decreased by about 63% after the durability test, whereas in Example 1, the peak area appearing near 0.8 V vs RHE decreased only by 140; in Example 2, the peak area appearing near 0.8 V vs RHE decreased only by 19%; in Example 3, the peak area appearing near 0.8 V vs RHE decreased only by 120; and in Example 4, the peak area appearing near 0.8 V vs RHE decreased only by 31%. The integral value of this peak is proportional to the surface area of the platinum catalyst and as the platinum catalyst deteriorates and the particle diameter of the platinum nanoparticles increases, this integral value decreases. This suggests that the durability is improved in the present invention. FIG. 13 shows a change in the peak area values before and after the durability tests (peak area value after the durability test/peak area value before the durability test) regarding the platinum catalysts of Examples 1, 2, 3, and 4 and Comparative Example 1.

From the above, it can be understood that the durability of the battery can be improved by blending a melamine compound or a polymer including the melamine compound as a monomer in the electrolytic solution.

### INDUSTRIAL APPLICABILITY

The electrolyte and oxidation inhibitor of an electrode electrochemical oxygen reduction catalyst of the present invention can be used as an electrolyte for fuel cells and metal-air batteries.

## Claims

1. An electrolyte comprising a melamine compound and/or a polymer comprising the melamine compound as a monomer.

2. The melamine compound according to claim 1, wherein the melamine compound is represented by a general formula (1): 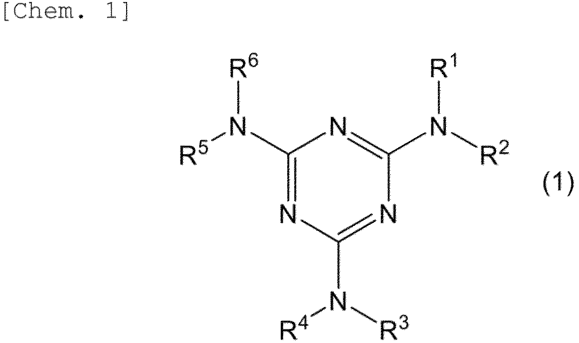 [in the formula, R¹, R², R³, R⁴, R⁵, and R⁶ are the same or different and each represents a hydrogen atom, a hydroxy group, a halogen atom, a substituted or unsubstituted alkyl group, or a substituted or unsubstituted alkenyl group].

3. The electrolyte according to claim 1, wherein the electrolyte is an electrolytic solution.

4. The electrolyte according to claim 3, wherein the concentration of the melamine compound and/or the polymer comprising the melamine compound as a monomer is 0.1 to 100 µmol/L.

5. The electrolyte according to claim 1, wherein the electrolyte is an electrolyte for a fuel cell or a metal-air battery.

6. An oxidation inhibitor of an electrode electrochemical oxygen reduction catalyst comprising a melamine compound and/or a polymer comprising the melamine compound as a monomer.

7. The oxidation inhibitor according to claim 6, wherein the melamine compound is represented by the general formula (1): 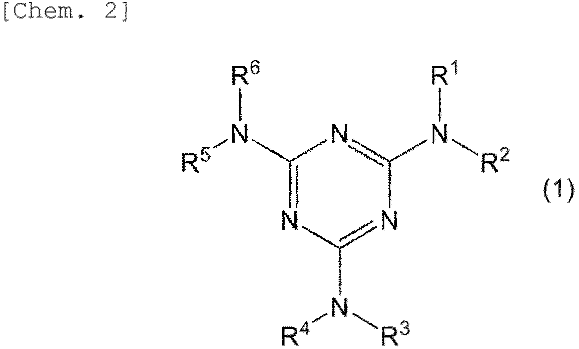 [in the formula, R¹, R², R³, R⁴, R⁵, and R⁶ are the same or different and each represents a hydrogen atom, a hydroxy group, a halogen atom, a substituted or unsubstituted alkyl group, or a substituted or unsubstituted alkenyl group].

8. The oxidation inhibitor according to claim 6, wherein the oxidation inhibitor is the melamine compound and/or a solution comprising the melamine compound.

9. The oxidation inhibitor according to claim 8, wherein the concentration of the melamine compound and/or the polymer comprising the melamine compound as a monomer is 0.1 to 100 µmol/L.

10. The oxidation inhibitor according to claim 6, wherein the electrode electrochemical oxygen reduction catalyst comprises platinum nanoparticles.

11. The oxidation inhibitor according to claim 6, wherein the electrode electrochemical oxygen reduction catalyst is a cathode catalyst for a fuel cell.

12. A fuel cell comprising the electrolyte according to any one of claims 1 to 5 or the oxidation inhibitor according to any one of claims 6 to 11.

13. A metal-air battery comprising the electrolytic solution according to any one of claims 1 to 5 or the oxidation inhibitor according to any one of claims 6 to 11.
